# EUROPEAN PATENT SPECIFICATION

(11) **EP 1 905 468 B1**
(45) Date of publication and mention of the grant of the patent: **07.11.2012**
(21) Application number: 06425670.4
(22) Date of filing: 29.09.2006
(51) Int. Cl.: A61M 11/00

(54) **Aerosol apparatus, in particular for children**
Aerosolgerät, insbesondere für Kinder
Appareil pour délivrer un aérosol, spécialement pour enfants

(43) Date of publication of application: 02.04.2008
(73) Proprietor: Zambon S.p.A., 20091 Bresso MI (IT)
(72) Inventor: Zambon, Elena, Zambon S.p.A., 20122 Milano (IT); Basso, Gianluca, Zambon Group S.p.A., 20091 Bresso (MI) (IT)
(74) Representative: Colombo, Stefano Paolo

(56) References cited:
- EP-A1- 0 667 168
- US-A1- 2004 210 151
- US-B1- 6 442 018

## Description

The present invention relates to an aerosol apparatus for administering a substance (typically a medicinal substance) by means of inhalation, in particular (but not exclusively) to a child.

It is known that the inhalation of air in which curative substances are dispersed has a therapeutic effect on infections of the respiratory apparatus. In addition to natural nebulization sources (for example, standing close to the sea allows one to breathe iodine which is nebulized by breaking of the waves) fairly basic nebulization methods such as vapour inhalations are known. Aerosol nebulization apparatus, or, in short, aerosol apparatus, are also known.

An aerosol apparatus allows the introduction, into the respiratory apparatus, by means of inhalation, of a substance (typically a medicinal substance) in the "sol" state for therapeutic purposes. The "sol" state is the state of a material which is midway between that of a liquid and gas. In other words, the particles which form it are not as dispersed as those of a gas, but are also not as concentrated as those of a liquid substance.

Aerosol nebulization is a therapeutic technique which is very advantageous because it favours the administration, within the respiratory tract, of drugs which may exercise their pharmacological effect *in situ.*

Aerosol apparatus of the mechanical (or pneumatic) type are known. They consist of a membrane or piston compressor and an actual nebulizer. Nebulization is obtained by means of the Venturi effect: the compressor produces an air flow at a high pressure and high speed which is forced onto the end of a sample vessel containing the medicinal substance. The liquid is then "atomized" into particles which may be inhaled through the mouth or nose, or both, by means of a special mask.

Ultrasound aerosol apparatus in which nebulization is achieved by means of a piezoelectric effect are also known: a transducer emanates electromagnet waves of the ultrasound type which fragment the medicinal solution.

Whatever the type of aerosol apparatus used, the methods of performing the aerosol therapy in order to achieve a good therapeutic result are very important. For the same amount of medicinal substance inhaled, the effectiveness of the aerosol therapy depends essentially on the length of time during which the patient undergoes therapy and the respiration frequency. In other words, treatment by means of aerosol therapy is particularly effective if the patient inhales the medicinal substance in the sol state for a period of about 15-20 minutes (or also more) and if inhalation is performed breathing deeply and at regular intervals.

However, many patients do not willingly undergo aerosol therapy sessions and, of their own initiative, tend to reduce the inhalation times, i.e. very often the patients interrupt inhalation before the end of the predefined time period considered to be optimal. Moreover, some patients tend to experience negatively or are even anxious about the aerosol therapy session. This causes them to breathe in an irregular, fast and superficial manner.

In all cases, the reduction in the time and the irregular breathing do not allow optimum therapeutic results to be obtained.

The abovementioned problems generally arise more frequently in the case of patients who are children. It has been noted, in this connection, that more than 50% of the patients who are children perform aerosol therapy incorrectly and discontinuously, resulting in no beneficial therapeutic effect. Young patients are often unable to understand the benefits which can be achieved with an aerosol therapy session and do not willingly agree to use an aerosol apparatus. In the case of very young children it is not even possible to convince them that it is better to take deep breaths and to remain wearing the mask for a certain predetermined period of time.

Various manufacturers of aerosol apparatus, in an attempt to solve at least partly the abovementioned problems, aim to provide apparatus which are able to perform increasingly shorter aerosol therapy cycles.

US 6,962,151 describes an electronic nebulizer which allows the therapy period to be reduced considerably to about three minutes.

The Applicant has faced the abovementioned problems in an attempt to provide an aerosol apparatus which is able to ensure more effective therapy.

The Applicant has found that the primary factors which make an aerosol therapy treatment effective are the frequency and the intensity of breathing. The Applicant has also noted that said factors, in turn, depend on the state of agitation or relaxation of the patient: a person who is anxious and not relaxed takes short quick breaths at relatively frequent intervals; on the other hand, a relaxed patient generally breathes more deeply and at a slower rate. The latter is a preferable condition for performing aerosol therapy.

Another factor considered to be important for the purposes of obtaining the improved benefits from an aerosol therapy session is the need to keep the patient's face in contact with the inhalation mask. If the patient moves his/her mouth and/or his/her nose away from the mask, the substance in the sol state is mixed with air and results in "dilution" of the inhaled substance, in addition to cooling and condensation.

EP 0 667 168 discloses an inhalation training device for maximizing the delivery of an airborne medication to receptor sites in a patient's lungs. It comprises inhaler means including a source of airborne medication for inhalation by a patient, a flow measurement means coupled with the inhaler means for measuring the flow of air through the inhaler means, a microprocessor means for integrating the flow of air over a time to produce a measure of volume inhaled, storage means for storing the measured flow and volume values and for storing additional quantifiable diagnostic parameters related to projected patterns of medication distribution to receptor sites in a patient's lungs, and display means for providing a visual display of measured flow and volume values of the patient as well as said diagnostic parameters related to projected delivery of medication to receptor sites in the lungs of the patient and for contrasting measured values with desired levels for those values.

US 2004/210151 discloses a respiratory monitoring, diagnostic and therapeutic system including a mask apparatus fitted with a pH sensor.

The object of the present invention is therefore to provide an aerosol apparatus which solves the abovementioned problems and in particular ensures that the patient is in the best psychological and physical condition to undergo aerosol therapy, resulting in completely effective treatment.

This object, together with others, is achieved by an apparatus having the characteristic features of Claim 1. Further advantageous characteristics of the invention are contained in the dependent claims.

An advantage of the invention is that the display device may be able to be separated from the apparatus so that it is possible to use it separately.

The apparatus advantageously is suitable for use by children. A detailed description of the invention now follows, being provided solely by way of a non-limiting example, to be read with reference to the accompanying figures in which:
- Figure 1 shows in schematic form an aerosol apparatus according to an embodiment of the invention, while it is being prepared for use; and

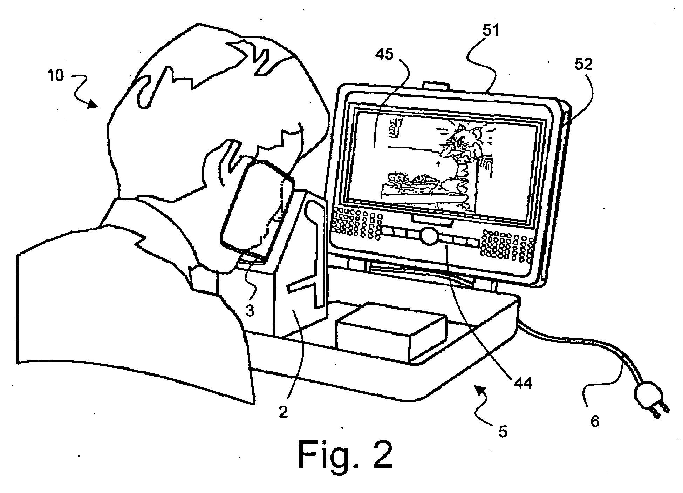

- Figure 2 shows in schematic form the aerosol apparatus according to Figure 1 during use.

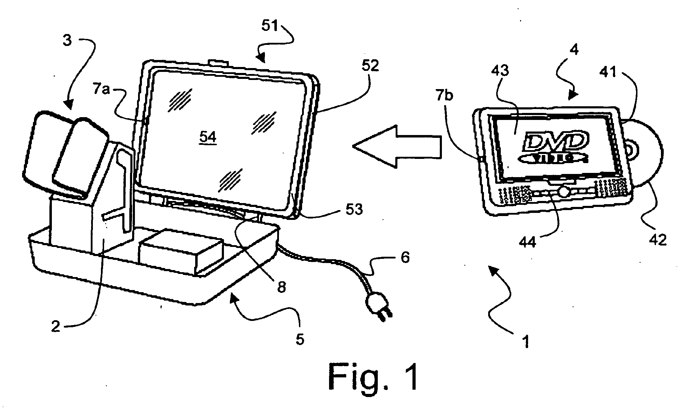

An aerosol apparatus 1 according to an embodiment of the present invention is schematically shown in Figure 1. The apparatus 1 comprises a nebulizer device 2 and a mask 3. The nebulizer device 2 may be equally well of the mechanical, pneumatic, ultrasound or any other type. The mask 3 is connected to the nebulizer device 2 so as to receive a substance (for example a medicinal substance) substantially in the sol state. During use, the mask is moved towards the mouth and/or the nose of the patient who is thus able to inhale the substance which is typically for therapeutic purposes.

According to the present invention, the apparatus 1 also comprises a display device 4 which allows sequential viewing of images, i.e. a succession of one or more static and/or moving images. The multimedia display device 4 may comprise a monitor of a computer (typically a portable computer), mobile phone (for example smart phone), so-called palm-top computer or the like. The display device 4 may conveniently comprise a television monitor. Even more preferably, the display device 4 may comprise a player (and/or recorder) of magnetic and/or optical media such as a DVD or video CD or videocassette player (and/or recorder).

In an advantageous embodiment, the display device 4 comprises a DVD player. In another advantageous embodiment, the display device 4 comprises a monitor of a personal computer or the like for displaying educational games or the like.

Typically, the display device 4 comprises means for emitting sounds associated with the images displayed. Therefore, for example, in the case where a film or a cartoon is shown, the images will be accompanied by the corresponding audio or sound signals.

In addition or as an alternative to the abovementioned sound emitting means, the display device may comprise means for displaying writing associated with the images shown. Therefore, for example, in the case where a film or a cartoon is shown, the images will be accompanied by subtitles. This function is useful for users who are deaf or have impaired hearing or when it is required to avoid disturbing people who are in the vicinity of the aerosol apparatus (but not involved in the therapy). This function is also useful where the audio associated with the images is in a language different from the native tongue of the user. In this case, the therapy session could also be used to learn a new language.

According to an advantageous embodiment, the DVD player is of the type with slot-in (lateral) loading of the DVD discs. This type of player, as opposed to players with a hinged opening system, is formed by a single body of the parallelepiped type which is relatively thin (currently, for example, in the region of a few centimetres). A slot for inserting the discs is provided on the side of the parallelepiped body. A display monitor is instead present on a face of the player. Operating keys may also be provided laterally or on the face of the monitor. The abovementioned sound-emitting means (typically associated with the images displayed) may also be provided laterally or on the face of the monitor.

By way of example, the monitor may be conveniently of the LCD or plasma type. A size for the monitor which is considered suitable for the purpose is 7" with a side ratio of 16:9. Obviously, monitors with greater or smaller dimensions, if necessary with a different side ratio, may also be used.

According to an advantageous embodiment of the invention, the apparatus 1 comprises a case-like container for storing and transporting at least the nebulizer device and the mask. The case-like container has a closed bottom, side walls and a lid which may also be closed. The lid is hinged on a side wall (typically on the rear side wall). The lid may also be designed to house the display monitor. For example, a slit with a size such that the display monitor may be inserted inside it (for example the DVD player) may be envisaged.

The slit is conveniently provided on one side, taking into account, if necessary, the need to have access to the disc insertion slot so as to be able to introduce/extract easily a disc into/from the player when the latter is inserted inside the slit and is substantially housed inside the lid of the container.

The slit is conveniently delimited by the outer surface of the lid and by an inner rim. The rim defines a window with dimensions preferably at least equal to the dimensions of the monitor. Preferably, if the controls of the display device are situated on the front, the dimensions of the rim must not prevent axis to the controls.

Fixing means are envisaged for preventing the possibility of the display monitor inadvertently and involuntarily coming out of the seat.

The display device may, if necessary, be extracted from the slit which houses it so as to be able to use it independently of the nebulizer device. In other words, the display device (for example the DVD player) may also be used when the nebulizer device is not used.

The display device may be powered by a battery, for example a rechargeable lithium battery and/or by a separate electric power supply and/or by the same electric power supply which powers the nebulizer device. This latter advantageous possibility may be provided by envisaging a system of electrical contacts (or if necessary a plug and socket system) between the lid and the display device. The electric power is conveyed to the lid, for example via the hinges, in the same way as in a portable computer.

The distance and the inclination of the display device are critical for the invention. They are preferably calculated depending on the inhalation mask so that the best view of the images shown on the monitor is obtained when the patient's face is kept properly in contact with the mask. In other words, the position, distance and inclination of the monitor are a consequence of the position and the shape of the mask. The patient will not be encouraged to change position because this would worsen the view of the images shown on the monitor.

Conveniently, the mask 3 is shaped so that the vapour emitted does not disturb viewing of the images shown on the monitor.

The aerosol apparatus 1 has proved to be particularly effective when used for aerosol therapy carried out on a group of children. During inhalation cartoons were shown. All the children undergoing therapy reacted positively to the said therapy, maintaining without difficulty a correct position for the entire duration (about 15 to 20 minutes) of each single treatment cycle. For this purpose, the duration of the cartoons sequences shown was specially selected according to the duration of the treatment cycle. The degree of relaxation of the patients was calculated as a function of the frequency of closing of the eyelids (number of eye blinks per minute). A substantial reduction in the frequency of closing of the eyelids in patients concentrating on watching the images shown on the monitor was noted. It is considered that this state of relaxation was decisive in ensuring the effectiveness of the therapy, having positively influenced the patients' breathing.

## Claims

1. Aerosol apparatus (1) comprising a nebulizer device (2), a mask (3) suitable for receiving a substance substantially in the sol state from the nebulizer device (2), and it also comprises a display device (4) able to display a sequence of images to a patient while he/she is using the apparatus (1), **characterized in that** said aerosol apparatus (1) comprises a case-like container (5) having a closed bottom, side walls and a lid (51), wherein the lid (51) is hinged (8) on a rear side wall of said case-like container (5), wherein the lid (51) is designed to house the display device (4), wherein the lid (51) is inclined with respect to the closed bottom, wherein the inclination of the lid (51) about hinge (8) is calculated depending on mask (3) so that the best view of the sequence of images shown on the display device (4) is obtained when the patient's face is kept properly in contact with the mask (3).

2. Aerosol apparatus (1) according to claim 1, **characterized in that** said display device (4) displays images comprising entertainment images.

3. Apparatus (1) according to claim 2, **characterized in that** said sequence of images comprises a cartoon, a film or an educational game.

4. Apparatus (1) according to Claim 1, **characterized in that** said display device (4) comprises an optical or magnetic medium player.

5. Apparatus (1) according to Claim 1, **characterized in that** said display device (4) comprises a digital versatile disc, DVD, player or
a monitor of a personal computer.

6. Apparatus (1) according to any one of the preceding claims, **characterized in that** said display device (4) comprises a display or monitor comprising a liquid crystal display, LCD, or a plasma screen.

7. Apparatus (1) according to Claim 5 or 6, **characterized in that** said display device (4) is of the type with side loading of the digital versatile discs, DVDs.

8. Apparatus (1) according to any one of the preceding claims, **characterized in that** said display device comprises means for emitting sounds associated with the images displayed.

9. Apparatus (1) according to Claim 1, **characterized in that** said lid is provided with a slit having dimensions suitable for being able to introduce the display device inside it.

10. Apparatus (1) according to claim 9, **characterized in that** said display device (4) can be separated from the apparatus (1) so that it is possible to use it separately.

11. Apparatus (1) according to any one of the preceding claims, **characterized in that** the mask (3) is shaped so that the vapour emitted does not disturb viewing of the images of the display device (4).

## Patentansprüche

1. Aerosolvorrichtung (1), die ein Vernebelungsgerät (2) und eine Maske (3), die dazu geeignet ist, eine Substanz, die im Wesentlichen im Sol Zustand vorliegt, von dem Vernebelungsgerät (2) aufzunehmen, umfasst und die auch eine Anzeigeeinheit (4) umfasst, die einem Patienten eine Abfolge von Bildern anzeigen kann, während er/sie die Aerosolvorrichtung (1) verwendet, **dadurch gekennzeichnet, dass** die Aerosolvorrichtung (1) einen kastenartigen Container (5) mit einem geschlossenem Boden, Seitenwänden und einem Deckel (51) umfasst, wobei der Deckel (51) an einer hinteren Seitenwand des kastenartigen Containers (5) angelenkt (8) ist, wobei der Deckel (51) dazu ausgebildet ist, die Anzeigeeinheit (4) aufzunehmen, wobei der Deckel (51) gegenüber dem geschlossenen Boden geneigt ist, wobei die Neigung des Deckels (51) um ein Scharnier (8) abhängig von der Maske (3) ermittelt wird, so dass die beste Sicht auf die Abfolge von Bildern, die auf der Anzeigeeinheit (4) angezeigt wird, erlangt wird, wenn das Gesicht des Patienten in gutem Kontakt mit der Maske (3) ist.

2. Aerosolvorrichtung (1) nach Anspruch 1, **dadurch gekennzeichnet, dass** die Anzeigeeinheit (4) Bilder anzeigt, die Bilder zur Unterhaltung umfassen.

3. Vorrichtung (1) nach Anspruch 2, **dadurch gekennzeichnet, dass** die Abfolge der Bilder einen Cartoon, einen Film oder ein erzieherisches Spiel umfasst.

4. Vorrichtung (1) nach Anspruch 1, **dadurch gekennzeichnet, dass** die Anzeigeeinheit (4) ein optisches oder magnetisches Medienabspielgerät umfasst.

5. Vorrichtung (1) nach Anspruch 1, **dadurch gekennzeichnet, dass** die Anzeigeeinheit (4) ein Abspielgerät für Digital Versatil Discs (DVD) oder einen Monitor eines persönlichen Computers umfasst.

6. Vorrichtung (1) nach einem der vorhergehenden Ansprüche, **dadurch gekennzeichnet, dass** die Anzeigeeinheit (4) eine Anzeige oder einen Monitor umfasst, die oder der eine Flüssigkristallanzeige, LCD oder einen Plasma Bildschirm umfasst.

7. Vorrichtung (1) nach Anspruch 5 oder 6, **dadurch gekennzeichnet, dass** die Anzeigeeinheit (4) derart ausgebildet ist, dass die Digital Versatile Discs, DVDs, an der Seite eingelegt werden.

8. Vorrichtung (1) nach einem der vorhergehenden Ansprüche, **dadurch gekennzeichnet, dass** die Anzeigeeinheit Mittel zum Abgeben von Tönen, die mit den angezeigten Bildern zusammenhängen, umfasst.

9. Vorrichtung (1) nach Anspruch 1, **dadurch gekennzeichnet, dass** der Deckel einen Spalt aufweist, der geeignete Abmessungen zur Aufnahme der Anzeigeeinheit im Inneren desselben aufweist.

10. Vorrichtung (1) nach Anspruch 9, **dadurch gekennzeichnet, dass** die Anzeigeeinheit (4) von der Vorrichtung (1) getrennt werden kann, so dass die Anzeigeeinheit (4) einzeln verwendet werden kann.

11. Vorrichtung (1) nach einem der vorhergehenden Ansprüche, **dadurch gekennzeichnet, dass** die Maske (3) derart geformt ist, dass der abgegebene Dampf das Betrachten der Bilder auf der Anzeigeeinheit (4) nicht stört.

## Revendications

1. Appareil de distribution d'aérosol (1) comprenant un dispositif de nébulisation (2), un masque (3) approprié pour recevoir une substance sensiblement dans l'état de sol du dispositif de nébulisation (2), et comprenant également un dispositif d'affichage (4) capable d'afficher une séquence d'images à un patient tandis qu'il utilise l'appareil (1), **caractérisé en ce que** ledit appareil de distribution d'aérosol (1) comprend un contenant en forme de boîte (5) comportant un fond fermé, des parois latérales et un couvercle (51), dans lequel le couvercle (51) est articulé (8) sur une paroi côté arrière dudit contenant en forme de boîte (5), dans lequel le couvercle (51) est conçu pour loger le dispositif d'affichage (4), dans lequel le couvercle (51) est incliné par rapport au fond fermé, dans lequel l'inclinaison du couvercle (51) autour de l'articulation (8) est calculée en fonction du masque (3) de sorte que la meilleure vue de la séquence d'images présentée sur le dispositif d'affichage (4) soit obtenue lorsque le visage du patient est maintenu correctement en contact avec le masque (3).

2. Appareil de distribution d'aérosol (1) selon la revendication 1, **caractérisé en ce que** ledit dispositif d'affichage (4) affiche des images comprenant des images récréatives.

3. Appareil (1) selon la revendication 2, **caractérisé en ce que** ladite séquence d'images comprend un dessin animé, un film ou un jeu éducatif.

4. Appareil (1) selon la revendication 1, **caractérisé en ce que** ledit dispositif d'affichage (4) comprend un lecteur de support optique ou magnétique.

5. Appareil (1) selon la revendication 1, **caractérisé en ce que** ledit dispositif d'affichage (4) comprend un lecteur de disque numérique universel, DVD, ou un moniteur d'un ordinateur personnel.

6. Appareil (1) selon l'une quelconque des revendications précédentes, **caractérisé en ce que** ledit dispositif d'affichage (4) comprend un afficheur ou un moniteur comprenant un afficheur à cristaux liquides, LCD, ou un écran à plasma.

7. Appareil (1) selon la revendication 5 ou 6, **caractérisé en ce que** ledit dispositif d'affichage (4) est du type avec chargement latérale des disques numériques universels, DVD.

8. Appareil (1) selon l'une quelconque des revendications précédentes, **caractérisé en ce que** ledit dispositif d'affichage comprend des moyens pour émettre des sons associés aux images affichées.

9. Appareil (1) selon la revendication 1, **caractérisé en ce que** ledit couvercle est pourvu d'une fente ayant des dimensions appropriées pour permettre l'introduction du dispositif d'affichage à l'intérieur de celle-ci.

10. Appareil (1) selon la revendication 9, **caractérisé en ce que** ledit dispositif d'affichage (4) peut être séparé de l'appareil (1) de sorte qu'il soit possible de l'utiliser séparément.

11. Appareil (1) selon l'une quelconque des revendications précédentes, **caractérisé en ce que** le masque (3) est formé de sorte que la vapeur émise ne perturbe pas la vue des images du dispositif d'affichage (4).
